# EUROPEAN PATENT SPECIFICATION

(11) **EP 3 021 736 B1**
(45) Date of publication and mention of the grant of the patent: **13.05.2020**
(21) Application number: 14733676.2
(22) Date of filing: 20.05.2014
(51) Int. Cl.: A61B 5/00

(54) **CONTACT SENSOR**
KONTAKTSENSOR
CAPTEUR DE CONTACT

(30) Priority: 22.05.2013 GB 201309260
(43) Date of publication of application: 25.05.2016
(60) Divisional application: 20168928.8
(73) Proprietor: Nextlife Wearables Ltd., London EC4N 7BP (GB)
(72) Inventor: PEDLEY, Mark, Wilmslow Cheshire SK9 6BG (GB); MCCARTHY, Ben, Lancaster LA1 4TA (GB); WARD, Adrian, Barrow-Upon-Soar Leicestershire LE12 8LW (GB); BAKER, Andrew, Wilmslow Cheshire SK9 4EP (GB)
(74) Representative: Stratagem IPM Limited
(86) International application number: PCT/GB2014/051539
(87) International publication number: WO 2014/188171

(56) References cited:
- WO-A1-2011/030099
- WO-A2-2009/013704
- US-A1- 2008 271 999
- US-A1- 2009 203 984
- US-A1- 2013 116 532

## Description

### FIELD OF THE INVENTION

The present invention relates to contact sensors and more specifically, although not exclusively, to the field of smart garments or wearables, in particular wearable items that contain physiological or biometric sensors as an integrated part of the wearable.

### BACKGROUND OF THE INVENTION

Such items often include a means of collecting the data generated by the sensors and, further, transmitting such data to remote devices for analysis. Various prior art devices, systems and methods have been proposed for such smart wearables, but these all have limitations. What is required is an element or set of elements that allow a comfortable wearable that is low cost, washable, and wearable for extended periods of time without inconvenience to the user. Wearables may include any garment or other item that may be worn on the human or animal corpus, such as clothing, hats, shoes, socks, belts, etc, as well as wrist/ankle bands, or bandages or blankets, etc.

Sensors for monitoring activity at a body surface are required to make intimate contact with the surface in order to function effectively. Prior art sensors have previously assured such contact by adhering a sensor to a skin surface, or at least interposing a layer of conductive jelly to ensure the transmission of signals from the body of the sensor, or even dampening the sensor and/or skin so as to provide a moisture layer for the same purpose. Some prior art research has been directed towards the attachment or incorporation of sensors on garments, with a respective garment holding a sensor in place.

An improvement on the prior art, a 'contact sensor' has previously been described in UK patent GB2444203 (Dias et al), comprising a contact membrane, a cover membrane elastic relative to the contact membrane; and a support element over which the cover membrane is stretched, with the contact membrane being attached to the cover membrane at the periphery of the element, such that at least one of the membranes forms a convex outer surface, the cover membrane being adapted for extension over a body surface to project the contact membrane against a body surface beneath it. WO2011/030099 discloses a compression sleeve formed from one or more knitted layers and comprising one or more electronic sensors for measuring signals from a wearer's body.

The prior art contact sensor essentially comprises two layers of fabric, each, in some embodiments, of varying elasticity, with a gap in between. One of the layers comprises conductive fibres. Between the layers lies a support element, nominally planar but possibly curved, which urges the conductive fibre layer outwards so that it makes a good pressure contact with a body surface - said body surface nominally being a skin surface of a human or animal corpus. There may also be provided a 'filler' layer additionally between the support element and the conductive fibre layer. In alternative embodiments, the 'filler' layer may itself take the place and provide the function of the support element.

It is therefore a first non-exclusive object of the invention to provide an improved contact sensor that at least mitigates the issues associated with prior art devices.

### BRIEF SUMMARY OF THE INVENTION

The invention is a contact sensor as defined in claim 1, comprising a thicker localized area.

The thicker area of the at least one base layer is formed by the addition of an extra thread, which may be introduced to the base layer, e.g. during the knitting process by which it is manufactured. The extra thread is thicker than the native threads of the base layer.

The contact membrane comprises a knitted conductive fabric, wherein the contact membrane is a knitted conductive fabric of higher stitch density than the fabric of the at least one base layer.

Additionally or alternatively, the contact sensor may be produced as one item by means of 3D knitting.

The width of the contact sensor is preferably 10mm or less, more preferably 6mm or less and most preferably 3mm or less. The body-contacting length of the contact membrane is preferably at least 60mm, more preferably at least 100mm and most preferably at least 200mm.

The contact membrane may be formed with a raised surface, for example as described above, e.g. such that the contact membrane is narrow in width and/or forms part of a contact sensor. The narrow contact sensor may be arranged upon the surface of the base fabric layer such that more than one length of contact membrane is available in use, e.g. to form the electrical connection with the conductive pad.

Additionally or alternatively, the conductive pad of the further item may be biased towards the contact membrane or membranes, such as by means of a pressure membrane that may be disposed over the further item. The pressure membrane may comprise a layer of elastic fabric, which may be attached to the base fabric layer of the wearable, e.g. so as to form a pocket into which, in use, the further item may be inserted.

In some embodiments, an inner surface of the pocket is provided with a non-slip surface layer, e.g. so as to restrict movement of the further item within the pocket. In some embodiments, an outer surface of the further item is provided with a non-slip surface layer, e.g. so as to restrict movement of the further item within the pocket.

In an optional aspect of the invention, one of the contact membrane, base layer, base fabric layer or an additionally provided layer may be hydrophobic or impervious to moisture and/or arranged so as to prevent moisture contacting the contact membrane.

Additionally or alternatively, one of the contact membrane, base layer, base fabric layer or an additionally provided layer is hydrophilic or otherwise suitable for the transmission of moisture so as to actively introduce moisture, where present in use, to the contact membrane.

### BRIEF DESCRIPTION OF THE DRAWINGS

Figures 1a to 1d illustrate a contact sensor according to a first embodiment of the invention;
Figures 2 and 2a to 2c illustrate a contact sensor according to a second embodiment of the invention;
Figures 3a and 3b show a garment comprising the contact sensor of the invention;
Figure 4 is a closer view of the upper back portion of the garment of Figures 3a and 3b;
Figures 5a to 5c show an electronic unit in accordance with another embodiment of the disclosure;
Figures 6a to 6c show embodiments of an electronic unit in place in a textile termination pocket;
Figures 7a and 7b show an alternative embodiment of the textile termination pocket / electronic unit combination;
Figure 8 shows a number of alternative connection arrangements; and
Figure 9 shows a front and back view of a pair of running shorts comprising a contact sensor in accordance with the invention.

### DETAILED DESCRIPTION OF THE INVENTION

By way of description of a specific embodiment, the present invention will be described. A 3-layer fabric is knitted using 3D knitting techniques. There are two 'base' layers of fabric which form the main fabric element of a wearable fabric item, such as a t-shirt. A third layer comprises fabric of conductive yarn. By use of 3D knitting techniques, with which the skilled man will be familiar, the third layer may be knitted so that it may lie between the 'base' layers, or may lie on the top or bottom surface of the 3-layer fabric, outside the 'base' layers.

It will be known to those skilled in the art that the 3D method of flatbed knitting allows layers, such as in this case a conductive layer, to switch surfaces. In other words, the fabric may be knitted such that the conductive layer may be exposed on one side or the other of a fabric of at least two layers (the conductive layer forming one such layer), or, in the case of a fabric of at least 3 layers, may be woven so as to sit between any other 2 layers and be isolated from the external surface of the fabric. It may 'weave' between layer surfaces of the fabric, so as to allow for the creation of conductive areas such as contact sensors on external surfaces of the fabric and then create conductive pathways within the fabric for conducting signals picked up by such contact sensor areas in an insulated pathway.

In wearables according to the present invention, this will allow for comfort, electrical insulation of the conductive fibres where necessary, and visual design of a wearable for aesthetic purposes.

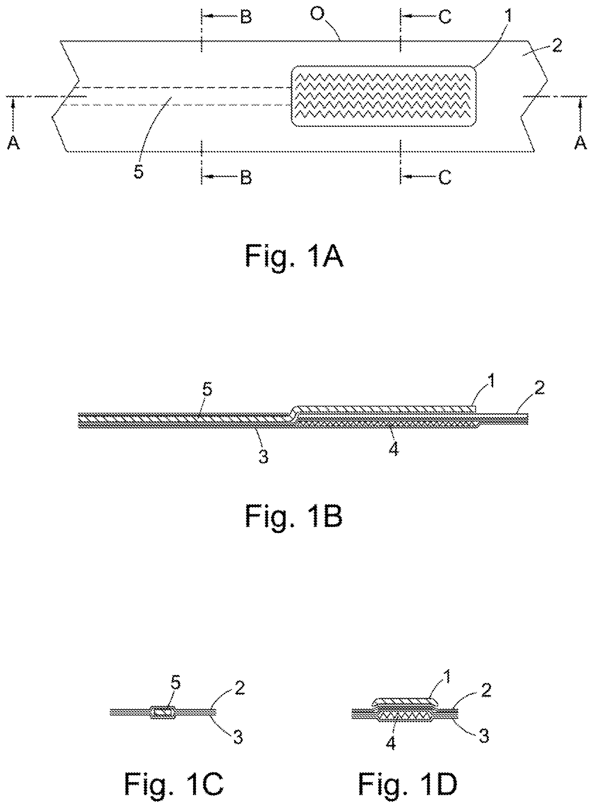

In the embodiment shown in Figures 1a to 1d, the conductive fabric layer is knitted into the form of a contact sensor body-contacting layer on one surface of the 3-layer fabric, and this is in connection with a comparatively narrow signal-conducting strip (5) of conductive fabric in between the two 'base' layers for the conduction of signals picked up by the contact sensor. Figure 1a shows the contact sensor in a plan view with the contact membrane on view; 1b shows the same sensor in cross-section through the plane AA; 1c shows it in cross-section BB, and 1d in cross-section CC.

In the embodiment in the Figures 1a to 1d shown, the contact sensor body-contacting layer (1) is shown as a discrete patch of approximately 50mm x 15mm, which is a typical size of sensor in the art. The stitch density of the body-contacting layer is higher than that of the base layer (2) against which it lies, so that there is a natural tendency for the body-contacting layer to form a convex surface for the contact sensor (0), as previously described in Dias et al's patent. Instead of providing a support element and/or a filler layer however, the outermost of the 'base' layers (3) is knitted, over an area substantially contiguous with the contact sensor area, with an extra thread which is introduced to the stitches locally during the knitting process. This extra thread is thicker and has greater bulk than the native thread of the base layers, so that the 'knitting in' of this extra thread results in a localised area of base fabric (4), contiguous with the area of the contact sensor body-contacting layer, which has a greater thickness.

The introduction of this additional yarn may be facilitated using additional or tailored flatbed knitting machine feeders. This greater thickness of the base layer at this local area allows for the contact sensor to be positively urged against a body surface as previously described in Dias et al, ie: by pressure generally applied by the base layers of the garment, which are generally elastomeric, but without the necessity for providing a support element or a filler.

This allows for greater ease of manufacture over the prior art sensor as well as lower cost due to fewer components.

In a further embodiment, it will be apparent that in order to give even greater efficacy to the pressure contact of the contact sensor, a support element and/or filler as per Dias et al could additionally be provided in addition to the locally thicker base fabric layer. The thicker base fabric layer (4), in the embodiment where there are two base layers (2, 3) of which the main fabric layer is constructed, could be either of said base fabric layers (2, 3). The area of thicker base fabric may be an area smaller or greater than the area of contact sensor, instead of a strictly closely matching size.

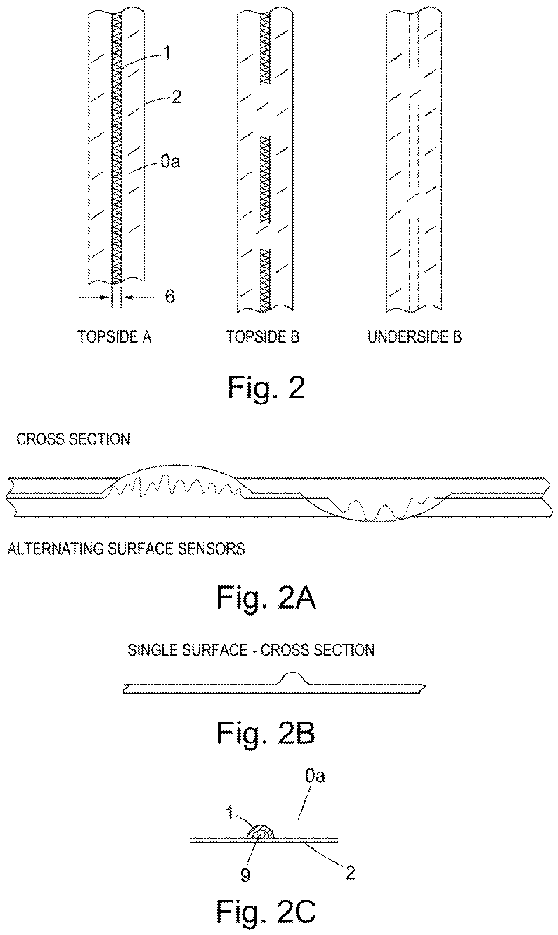

In a further aspect of the invention and as a preferred embodiment, the contact sensor is of an atypical narrow, elongate area form. Accordingly there is provided a contact sensor for monitoring activity at a body surface, comprising a contact membrane (1) having a body surface contacting area comprising knitted conductive fabric, and further comprising a base layer (2) of knitted fabric, and the knitted conductive fabric is of higher stitch density than the knitted fabric of the base layer, wherein the contact sensor is narrow in width such that the contact membrane forms a raised outer surface without the necessity for a support means. This is typified in Figure 2.

Prior art contact sensors typically have a surface area defined by at least a 15mm width and 30mm length, up to approximately 20mm width and 60mm length, which provides a size consistent with the requirement to pick up electrical signals from a discrete point on the body. In an aspect of the present invention, the contact sensor is comparatively elongate and narrow.

In Figure 2, it is shown as having a width (6) of approximately 3mm and an extended length extending beyond the portion of fabric shown. The preferred width range is in the region of 2mm to 10mm, which is generally congruent with the seams of garments. A typical normal clothing garment such as a t-shirt has a 2-6mm seam at fabric joins. Some specialist items of clothing such as skiing jackets have seams of up to 10mm width, consistent with the greater requirements for strength and weatherproofing expected of such garments.

Accordingly, preferred ranges are from 2-6mm or 2-10mm width for the preferential embodiments of the elongate contact sensor. In further preferential embodiments, the contact sensor has a length of greater than 60mm, greater than 100mm or even greater than 200mm.

Prior art contact sensors are usually of much lower lengths because, as previously inferred, it is desirable to 'pick' up electrical signals from relatively small discrete areas of the body to prevent signal interference. Normally a reading is only desired from a particular muscle or muscle group, and so a smaller sensor is preferred that can be located precisely upon that muscle or muscle group. Prior art sensors are directed towards picking up signals from the heart of a wearer.

There is the further mechanical limitation engendered by a requirement to site prior art electronic devices (data capture and/or signal analysis devices) within a short distance of prior art sensors, to minimise noise created by connecting wires and/or movement of the wearable itself - ie; folding or flexing of a garment in use. Standard prior art sensors still generally have a minimum size as previously noted, however, as they still need to be big enough to pick up a good signal, and require this minimum size in order to do so, and additionally normally require the further assistance of moisture via electrolyte conductive media.

Surprisingly, however, it has been found that the improved prior art contact sensor of Dias et al can be reduced in width to 10mm or less, 6mm or less, or even 3mm or less as noted and still pick up a good signal, with a dry sensor, due to the superior construction thereof. The further improvements noted as an aspect of the current invention, whereby a contact sensor similar to Dias et al is produced without support elements or fillers, capitalise on this as the sensor of Dias et al becomes increasingly difficult to manufacture as the width of the sensor decreases - the improved sensor of the current invention is simple to manufacture even in very small widths, as it requires no insertion of materials (support elements or fillers) between layers of fabrics. Thus the improved sensor can be readily manufactured in the smaller widths yet is capable of very good signal pick-up.

Even more surprisingly, a contact sensor as revealed in Dias et al, whereby a conductive layer is produced with a greater density of stitches than a base layer against which it is knitted, thus forming a convex surface which may form a contact surface amenable to creating a good contact with a skin surface just by pressure application, has been found, when produced in the narrower widths as noted for the current invention, to function particularly well and provide good signal pick-up even if no support element or filler (as per Dias et al), or even localised thickening of base layer (as per one aspect of the present invention), is present (Figure 2c). Further, it has been found that such a narrow or slim line reduced-width elongate contact sensor is not uncomfortable for a wearer in use.

With regards to the lengths of the sensor in an aspect of the present invention, when applied to a wearable item as a whole (garment, bandage, wrist or ankle band, etc), the usual disadvantage of a long sensor, as noted, is the risk of signal interference from more than, for example, one muscle or muscle group. Prior art sensors of the fabric type tend to be limited to picking up heart or pulse signals, so a fairly specific location is required. In an aspect of the present invention, the signal or signals are conducted along the length of the elongate sensor (0a) until they reach an electrical termination or connection point (7) at which the signal enters an electronic apparatus or unit (8).

As noted previously however the conductive layer, and thus in essence the elongate contact sensor, may be woven, over its length, by 3D knitting so as to sit on external surfaces of the overall fabric of a wearable or may be knitted in between other layers to as to be insulated from either or both the external environment or the body of a wearer. Accordingly the elongate contact sensor (0a) may not be in contact with the body of a wearer along the entirety of its length (although it may be, depending on the data collection task required), and may in fact be insulated from it, but the essence of the invention is not thus detracted from and the term elongate contact sensor or contact sensor is intended to cover such insulated lengths of the device despite said insulated lengths not strictly being 'in contact' at those points.

The electronic unit may be such as to perform one or more of various processing tasks upon the raw data (in the form of electrical signal from the contact sensor) that reaches it, or may simply be a transmission unit for transmitting the raw data onwards, possibly via wireless connection (radio (RF), Bluetooth, infra-red, NFC or various other known means) to further electronic or computing devices, which may be specialist devices or may be relatively general items such as smartphones running 'apps'.

In an aspect of the current invention, the raw data is processed by algorithm which is capable of identifying and isolating a desired signal or indeed plurality signals from other, unwanted or 'noise' signals that may be picked up by the elongate contact sensor. By way of example, the same elongate sensor may pick up heartbeat signals where it contacts the relevant area of a human or animal corpus whilst also picking up other muscle signals from pressure contact elsewhere on the same body - for example on a human subject, a length of elongate sensor might begin on the chest, where it picks up heartbeat signals, then may run to the seam of the arm of a garment, then run across the back of the subject, picking up signals from the latissimus dorsi and trapezius muscles - the algorithms of the electronic devices allow processing of the raw data from the elongate sensor so as to discretely measure heartbeat and/or other signals - in the case noted, for example, latissimus and trapezius contracture.

Such a combination may be, for example, particularly useful in the training of a rower, where the discrete packets of data may be used in analysis of rowing technique and/or measurement of the subject's efficiency, or exhaustion, or any other desired characteristic or state of the subject. The same could apply to other muscles and other animals as well as other forms of exercise or exertion, such as running for example, which may be particularly pertinent in the case of horses.

Advantageously, the elongate contact sensor can be knitted into garments in a manner known to those in the art, particularly as noted in patent WO2007036746 (also to Dias but also Hurley et al, 'Knitting Techniques'). Conveniently the elongate sensor can be made congruent with the seams of a garment for sections of its length, which aids in comfort, improves versatility in choosing collection sites on the wearer, the discreet placing of sensors, and manufacturability.

The contact sensor, as noted, comprises conductive fibres in the contact membrane surface. Prior art contact sensors are limited to measurement of heart rate. In another aspect of the present invention, the sensor is adapted to measure other physiological or biometric activity, such as temperature or moisture (sweat), or some or all of the conductive fibres may comprise part or all of a strain gauge (such as noted in WO2009093040, Dias/Hurley - 'Linear Electronic Transducer') for measuring motion directly by noting changes in the fabric of a wearable due to stretching or other motion of the fabric itself, instead of indirectly by measuring signals correlating to muscle contracture.

In one aspect of the invention, one of the layers of fabric, either a base layer (2, 3) of a garment/wearable, or the conductive contact sensor surface (1) itself, comprises a layer that is particularly hydrophobic or hydrophilic. In some circumstances, a hydrophobic material would be preferable to minimise the effects of moisture (ie; sweat, or possibly rain or other externally introduced moisture) on the sensor, by acting as a barrier to prevent the moisture reaching the conductive material, where, for example, such moisture would interfere with the picking up or transmission of an electrical signal. In another embodiment, a hydrophilic material might be used, such as for example where it is desirable to ensure moisture (ie: sweat) is transferred to the conductive layer, specifically to change the 'pick up' or transmission characteristic, or the conductivity of the material, so as to use the sensor to detect sweat or other moisture or amounts thereof.

In a further aspect of the current invention, it is possible to insert a further element (9) into the longitudinal 'gap' or 'space' in between the conductive contact sensor membrane and the one or more layers of 'base' fabric to which it is attached. Such further element may introduce further functionality into the wearable. It may comprise a capillary device. In one embodiment the element may be an optical fibre. Preferably such optical fibre may be used to transmit further physiological data along the path defined by the elongate sensor, in addition to the other signal or signals already being conducted by this route.

For example, a Doppler signal may be used to pick up a pulse signal at a suitable site, such as a wrist if the subject is human or similar sites on other animals. Advantageously an optical sensor may read a pulse through thin layers of clothing or other intermediate barriers, such as hair, which is particularly useful in the case of animals, ie: horses. A signal from such a sensor can be transmitted via an optical fibre or fibre capillary through a path defined by an elongate contact sensor.

As noted, in an aspect of the present invention and in various embodiments, signals are conducted along the length of the elongate sensor until they reach an electrical termination or connection point at which the signal enters an electronic apparatus or unit. In prior art devices, the connection between fabric based sensors and more traditional hardware is commonly a metallic two-part press-stud or snap-button connector, one part of which is fitted to the wearable, physically and permanently contacting the conductive fibres and requiring a manufacturing step to install, usually involving the creation of a hole in the fabric of the wearable, or using prongs or staples to puncture and grip the fabric. This arrangement has the disadvantages of extra manufacturing steps and extra cost, as well as potential comfort issues and possible impact on washability due to the presence of hard metal objects effectively embedded in a garment or other wearable.

Furthermore, this creates localised points of weakness in one or more surfaces of a garment or other wearable, which may lead to rips or tears. What is required is a means of effectively connecting soft fabric-based conductive material comprising part of a fabric wearable item to a metal or other conductive element of a separable item, which would preferably form part of or subsequently connect to further electrical or electronic components, without the disadvantages of the prior art methods.

Accordingly there is provided an electrical connection point (7) for a wearable item comprising a base fabric layer (2, 3), wherein the electrical connection point comprises: a contact membrane (1) comprising conductive yarn on a surface of the base fabric layer for electrical connection by means of pressure contact with a conductive pad (10) on an external surface of a further item (8) which is separable from the wearable item.

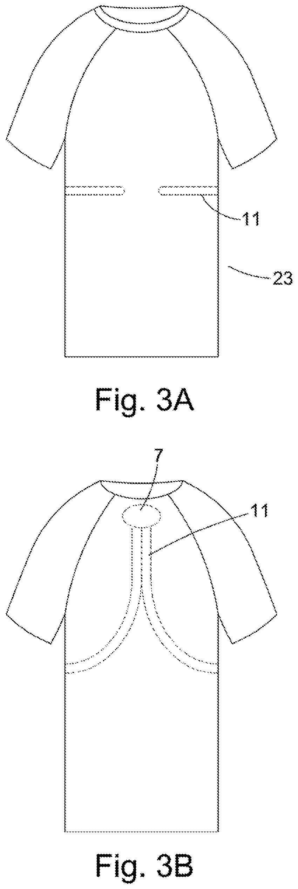

This aspect of the invention is shown in a preferential embodiment in Figures 3-6. Figure 3a shows the front of a t-shirt (23) in accordance with the present invention. The dotted lines (11) indicate the path taken by a pair of elongate sensors in accordance with one aspect of the invention. The contact membranes of the sensors are placed and knitted into the garment or pathways of the garment by 3D knitting so as to present lengths of contact membrane for contact with the chest of a wearer of the garment, and accordingly these lengths of the sensor are on the inner surface of the garment. By further 3D knitting, other lengths of the elongate sensor are isolated from the inner surface by one of two base fabric layers of the garment, and from the outside of the garment by the other base layer.

Figure 3b shows the back view of the t-shirt. The elongate sensor lengths run across and up the back of the garment, as again indicated by the dotted lines, up to an interface or 'textile termination point' (7) which in this case is located towards the upper part of the garment, near the neckline. On a subject wearing the garment, this would be roughly situated just below the base of the neck or the top of the spine, between the shoulder blades, which has advantageously been found to be a suitable position for the textile termination point, in particular from the point of view of comfort.

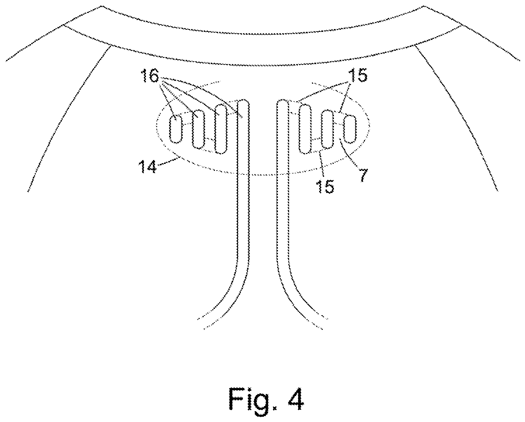

Figure 4 is a closer view of the upper back portion of the garment, and shows the arrangement of the elongate sensor as it traverses the rear side of the garment up to the interface/textile termination point. In this figure, the outermost layer of the garment is not shown so as to show the elongate sensors traversing the outward surface of the inner base fabric layer (ie: the surface of the inner base fabric layer that would not in use be contact with the skin of the wearer). Also not shown is the fabric layer (13) which comprises part of the textile termination pocket (12) which is a further aspect of the invention described later herein. The outer periphery of the textile termination pocket where it attaches to the base fabric layers is shown in this Figure 2 as a dotted line (14). It can be seen that the elongate contact sensors present a number of lengths of contact membrane - connection lengths - on the outermost surface of a layer of base fabric within the boundary of the textile termination pocket. Dotted lines (15) indicate where the elongate sensor is present on the inner surface of the base fabric layer, although in this case this is done partly for aesthetic value as of course the 'turns' at the ends of the connection lengths could be presented on the same surface as the connection lengths themselves.

As discussed previously herein, with regards to the elongate contact sensors, the contact membrane surfaces of the connection lengths preferably present convex surfaces. A substantially flat conductive element (10) placed or pressed against the convex surface of at least one of these connection lengths will result in a good electrical contact without the requirement for any of the disadvantageous prior art methods of connection as previously described. In this embodiment, a number of connection lengths (16) of elongate contact sensor being available means that the chances of good electrical contact are increased.

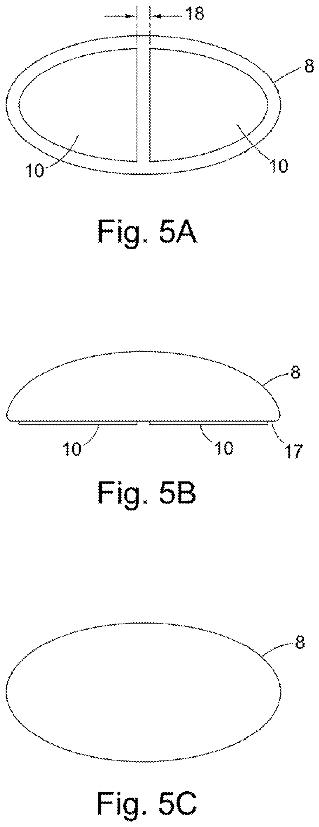

Figure 5a shows the underside of an electronic unit (8) in accordance with another aspect of the disclosure. It is provided with a pair of conductive elements (10) in the form of pads or shims. In the embodiment shown, these pads or shims are metal plates.

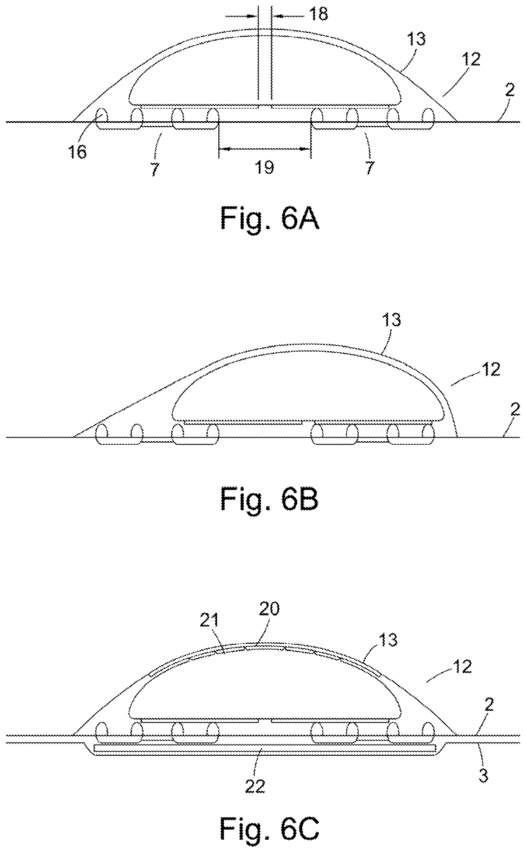

In use, the electronic unit is placed within the textile termination pocket (12) with the underside of the electronic unit (17) facing the sensor membrane surfaces of the textile termination points, such that the textile termination points are in contact with the pads of the electronic unit (Figures 6a to 6c).

It can be seen (Fig 6a) that the distance between the pads of the electronic unit (18) is small relative to the distance between the textile termination points (19). The electronic unit is intended to be a snug or tight fit within the textile termination pocket so that pressure between the pads and the textile termination points is maintained so as to maintain a good electrical connection. It can be seen that the textile termination pocket is of oval shape and of similar size to the oval planform shape and size of the electronic unit, so as to maintain the electronic unit in the pocket and in a particular orientation.

It is noteworthy that the electronic unit and textile termination pocket do not need to be oval in planform - alternative embodiments may be of rectangular or other planforms. The essence is that the shape would normally be chosen so as to provide for a complementary fit of pocket with electronic unit so that the electronic unit moves around in the pocket as little as practicable given the limitations of the materials of which the pocket is made, which are preferentially fabric so as to provide maximum comfort to the user rather than of harder materials or substances.

However in practice the electronic unit (8) will move around at least a small amount within the textile termination pocket, and so to prevent one of the pads (10) connecting to both textile termination points (7) simultaneously whilst in use, which would disrupt signal pick-up by the electronic unit, the larger gap (19) between the textile termination points (larger than that (18) between the pads of the electronic unit) allows for a degree of lateral movement of the electronic unit. Said potential movement of the electronic unit within the textile termination pocket is also a reason why the textile termination point comprises a number of parallel connection lengths (16) - lateral movement of the electronic unit does not result in a pad of the electronic unit becoming displaced from any possible electrical contact with the elongate sensor.

Figures 6a and 6b illustrate - viewed from above and in figurative cross-sectional view. Figure 6a shows an electronic unit in place in a textile termination pocket in a 'central' position and Figure 6b shows the same electronic unit displaced to one side. It can be seen that the pads of the electronic unit remain in contact with their respective textile termination points in the displaced position whilst the larger gap between the textile termination points, relative to the gap between the electronic unit's pads, prevent cross-connection.

In the normal, central position, the pads of the electronic unit are each in contact with 2 connection lengths of their respective textile termination points, whilst in the displaced position one pad is in connection (and thus electrical contact) with a single connection length whilst the other is in electrical contact with all four of the available connection lengths of its respective textile termination point. Preferentially, there is provided an outer fabric layer which comprises the other fabric surface of the textile termination pocket. More preferentially, this outer pocket fabric (13) is elastic and formed as a tight fit over the electronic unit when the unit is in place, so that it produces pressure on the electronic unit which urges the pads of the electronic unit towards the textile termination point and creates a good electrical contact.

Advantageously, this elasticity of the fabric outer layer and the tight fit also aids in minimising movement of the electronic unit in the pocket. Figures 6a and 6b show this, with a textile termination pocket comprising a base fabric layer which is one of the base fabric layers of the garment, and an outer fabric layer. This outer fabric layer may be an area of a further base fabric layer of the garment, introduced by 3D knitting during manufacture of the main body of the garment, or may be a separate layer which is stitched into position subsequently.

In further embodiments, exemplified in Figure 6c, a number of extra elements or features may be introduced to improve the efficacy of the textile termination pocket. To aid in minimising movement of the electronic unit in the pocket, the inner surface of the pocket fabric outer layer may be provided with a surface (20) which increases friction between that inner surface and the electronic unit when it is in place. This may be a layer added to the fabric of the pocket outer layer (as shown in Figure 6c) or may be intrinsically a part of the fabric. A suitable such surface may comprise silicon or raw spandex or latex.

The upper surface of the electronic unit may also be provided with a surface finish or additional layer (21), which may be complementary to any surface provided by the inner surface of the pocket outer layer. In the embodiment shown in Figure 6c, it is shown as having an upper surface with a surface finish comprising a number of raised ridges. One or all of these features, or variants thereof, will have the desired effect. Also shown in Figure 6c is an additional layer (22) which may be a stiffening member which locally stiffens the pocket area of the base layer of the pocket. This has the effect of providing a stable surface against which the electronic unit is ultimately urged by the elastic quality of the pocket outer layer.

Further, or alternatively, it may provide insulation of the inner 'runs' of the elongate contact sensor from the body of a wearer. It may be hydrophilic, or impervious to moisture, to prevent moisture (such as sweat) reaching any of the components which are conductive (ie: the pads of the electronic unit or the elongate contact sensor connection lengths / the textile termination point). A further base layer (3) is shown in this Figure 6c embodiment which may also act to insulate the electrical components from the corpus of the wearer.

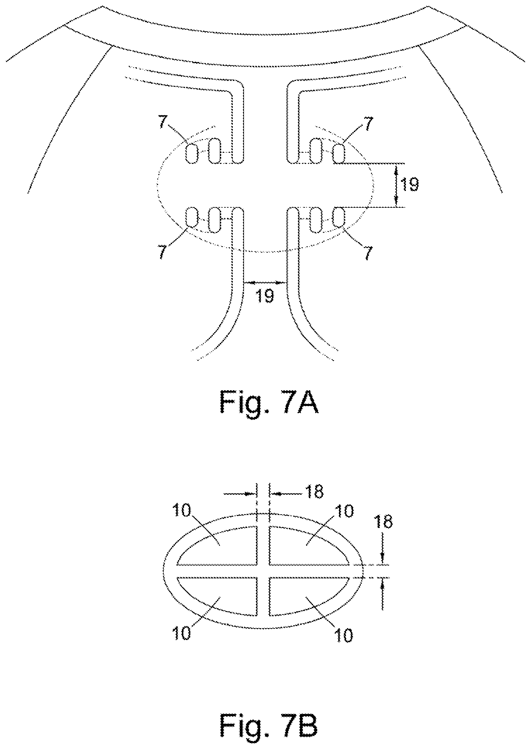

In an alternative embodiment of the textile termination pocket / electronic unit combination, (Figures 7a and 7b), the electronic unit is provided with four pads 10 and the textile termination pocket is provided with four textile termination points 7. Again, the distances between the textile termination points (19) are proportionally greater than those between the pads on the electronic unit (18) so as to allow movement of the electronic unit within the pocket whilst retaining an electrical connection of each pad with its respective textile termination point. Such an arrangement allows for the collection by a single electronic unit of data signals from four elongate contact sensors and thus proportionally more physiological or biometric data can be collected.

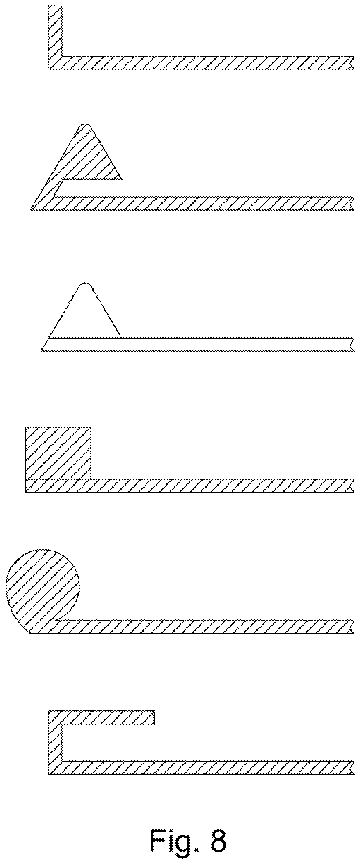

As can be seen in this variation the number of parallel connection lengths of each textile termination point is three rather than four - the number of such parallel connection lengths may of course be any integer from two upwards, or in fact a single length of contact sensor may suffice if the other elements of the textile termination pocket are sufficiently efficient as to allow for a good electrical contact. Neither need the connection lengths be parallel, nor even discrete - Figure 8 shows a number of alternative arrangements that would achieve the same desired effect, and the skilled man, presented with the essence of the invention, would readily be able to devise other variants which are considered to all fall within the ambit of the invention as claimed herein. In other possible embodiments, the number of pads on the electronic unit may be 3 or 5 or further integers, arranged in segments on the lower surface of the electronic unit as necessary to achieve the necessary spacing of the pads and textile termination points.

In a further aspect of the disclosure, the electronic unit (8) for use with the wearable is a 'smart' electronic unit, being a multi-function and multi-use device which is interoperable between different wearables. It contains a range of functionality for detecting and/or analysing and/or onwardly transmitting data that may be introduced to it originating from one or more sensors, which may be of differing types, such as contact sensors as otherwise herein described, linear electronic transducers, moisture sensors, temperature sensors or sensors for detecting other relevant physiological or biometric data. Preferably the data is introduced to the electronic unit via conductive pads or shims as previously described, which are, in use, in contact with textile termination points as also previously described.

By way of example, there may be two upper body garments (23); a first garment with elongate contact sensors for detecting EMG signals from upper body muscle groups and a second garment with elongate contact sensors for detecting heart rate signals. Each of the garments has two textile termination points located in a textile termination pocket, as described in another aspect of the invention. The electronic unit has two pads and contains circuitry for isolating and onwardly transmitting heart rate signals and also circuitry for isolating and performing analysis on EMG signals from a set of upper body muscle groups, then storing the analysed data. The electronic unit is initially in storage, such as in a drawer somewhere, and as it is receiving no inputs via the pads, it goes into 'rest' or 'hibernate' mode, which is a low-power mode in which it uses the minimum necessary battery power.

When the user places the electronic unit in the first garment and begins exercising (rowing for example), data signals of a particular pattern begin to enter the electronic unit. These data signals initiate a high-level circuit in the electronic unit, which recognises the pattern of data signal (in this case, data signals consistent with upper body activity) and activates the circuitry within itself for analysing and storing the signal data.

By way of alternative, the entire unit or parts thereof may 'wake up' upon the initiation of movement after a period of no movement, for example there may be an accelerometer within the unit, which produces a start-up signal upon movement of the unit, such as its removal from a drawer or shelf or suchlike. Having completed rowing, the user then removes the electronic unit from the first garment. Removed from the first garment, the electronic unit again becomes dormant, saving power. The user then inserts the electronic unit into the second garment and begins a different type of exercise (running, for example).

The electronic unit again recognises the type of signal (heart rate) and activates the 'transmit' circuitry within itself, and begins transmitting the raw data to a remote analysing unit. This may be, for example, transmission by Bluetooth to a smartphone which is running an app for analysing the heart-rate data.

Once the electronic unit is removed from the garment, it again becomes dormant and enters its low-power mode. In this way it can be seen that the electronic unit recognises the environment in which it is placed by virtue of the sensory input or inputs (or lack thereof) that it is receiving via the pads, and activates or deactivates relevant circuits accordingly.

This enables the unit to operate in as efficient a manner as possible and save battery power. It is also designed in this way so that the user is not required to perform any 'set up' actions prior to use - the user only needs to install the unit in a particular garment and the electronic unit does the rest. As an alternative or additional embodiment, the electronic unit may be required to fit into the textile termination pockets of different garments or other wearables in a particular orientation - an accelerometer on board the electronic unit is able to detect this orientation and this is a further means of identifying which set of sensors need to be activated, in accordance with the type of garment/wearable and the associated expected sensors.

This is particularly useful where a user may be short of time, perhaps due to a routine of different exercises. This is also a feature unique in this field, where wearables are routinely provided with a single set of dedicated circuitry which correspond to a single, or single set, of sensors, for detecting a single biometric parameter. For example, wearables of a 'wristwatch' type may measure pulse rate; a chest strap may measure heart rate.

A garment with an electronic unit able to measure more than one parameter is an aspect of this disclosure. An electronic unit that is interoperable between wearables, so that only one electronic unit need be purchased but which can then be moved between wearables, particularly of different types (ie: t-shirt, shorts, wrist strap), or between wearables where old ones may be thrown away and new ones bought, is a further aspect of the current invention.

It can readily be seen that this can provide significant cost savings, particularly over a situation as in the prior art where a wearable including its electronics is disposed of at the end of its useful life, or where several garments or other wearables each have their own set of electronics. As well as garments, wearables can include wrist or ankle bands, wristwatches, belts, bandages, shoes, or any other item worn on the human or animal body.

In a further refinement to this aspect of the disclosure, there is presented a collaborative software interface, which may again be in the form of a software 'app' for a smartphone, wherein the data from a number, ie; 2 or more, electronic units is collated. The electronic units may for example be installed in a number of different wearables, such as, for example, a pair of shorts (24), a t-shirt (23), a wristband device and a headband. The shorts and t-shirt may be set up with sensors to measure the contracture of various major muscle groups; the wristband may be set up to measure pulse rate, and the headband may comprise sensors to measure the presence of sweat. Given suitable algorithms, the collaborative software interface is able to provide full-metabolic overview of the body (ie: measurement of basal metabolic rate). As an alternative or additional mode, this collection of multiple subject inputs may allow for personalisation of an exercise programme.

In a further embodiment, the battery life of the electronic unit is extended due to use of Bluetooth 4. It is preferably also extended by maximising the amount of pre-processing of 'raw' data on the Electronic Unit itself, so as little data as necessary needs transmission to further analysis or user interface devices. In general, the biggest drain on batteries in such devices is data transmission, so this saves power.

In an embodiment of the present invention, there is provided a smart garment (24) for use on the lower body of a human subject, wherein said garment comprises a contact sensor as claimed in any of claims 1 to 11 for the detection of signals from the femoral artery.

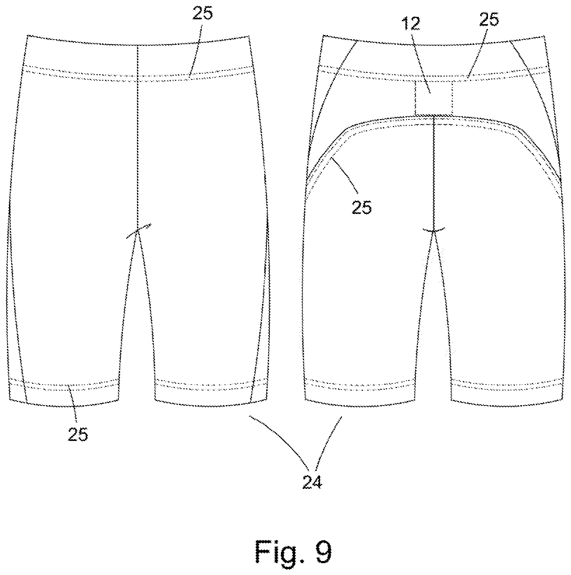

An embodiment of this is shown in Figure 9, which shows a front and back view of a pair of running shorts in accordance with the invention. Dotted lines indicate the paths (25) taken by elongate contact sensors through the shorts and a textile termination pocket (12) situated at a position towards the lower back of a wearer. Heart rate data is gathered by the elongate contact sensor in contact with the thigh, preferably the upper thigh, of the wearer, by picking up of signals from the femoral artery. There is generally provided, in the textile termination pocket whilst the garment is in use, a smart electronic unit in accordance with another aspect of the invention and as described herein, which is capable of isolating the heart rate signal from other signals and noise that may be picked up by the elongate sensor. Other signals may also be picked up by the elongate sensors, and subsequently enter the electronic unit for isolation, measurement, analysis, or transmission onwards, such as EMG measurements from various muscle groups, such as abdominal muscles, thigh muscles, or gluteal muscles. This can enable measurement of such characteristics as stride rate, work rate, or other characteristics of importance to the user.

## Claims

1. A contact sensor (0) for monitoring activity at a body surface, comprising:
a body contacting layer (1) of knitted fabric having a body surface contacting area; and
a base layer (2) of knitted fabric,
wherein the stitch density of the body contacting layer (1) is higher than that of the base layer (2) and wherein the base layer (2) is knitted with native threads of a first thickness (3), **characterized in that** the base layer is knitted with extra thread introduced to the base layer during the knitting process to form a localized area (4) that has a second thickness greater than the first thickness and the localized area (4) is congruent with the body surface contacting area of the body contacting layer (1) such as to urge the body contacting layer (1) into the forming of a raised outer surface for projection against a body surface.

2. A contact sensor (0) as claimed in claim 1, wherein the body contacting layer (1) is a knitted conductive fabric.

3. A contact sensor (0) as claimed in any preceding claim, wherein the contact sensor is produced as one item by means of 3D knitting.

4. A contact sensor (0) as claimed in claim 1, wherein the width of the contact sensor is 6mm or less.

5. A contact sensor (0) as claimed in claim 4, wherein the width of the contact sensor is 3mm or less.

6. A contact sensor (0) as claimed in any preceding claim, wherein the body-contacting length of the body contacting layer (1) is at least 60mm.

7. A contact sensor (0) as claimed in any preceding claim, wherein the body-contacting length of the body contacting layer (1) is at least 100mm.

8. A contact sensor (0) as claimed in any preceding claim, wherein there is further provided optical fibre (9) for the pick-up and transmission of Doppler signals from the body of a wearer.

9. A contact sensor (0) as claimed in claim 8, wherein the optical fibre is fitted within a channel formed between the body contacting layer and the base layer.

10. A contact sensor (0) as claimed in any preceding claim, wherein one of the body contacting layer (1), base layer (2) or an additionally provided layer (22) is hydrophobic or impervious to moisture and arranged so as to prevent moisture contacting the body contacting layer.

11. A contact sensor (0) as claimed in any of claims 1 to 9, wherein one of the body contacting layer, base layer or an additionally provided layer is hydrophilic or otherwise suitable for the transmission of moisture so as to actively introduce moisture, where present in use, to the body contacting layer.

## Patentansprüche

1. Kontaktsensor (0) zum Überwachen einer Aktivität an einer Körperoberfläche, umfassend:
eine Körperkontaktschicht (1) aus gewirktem Gewebe, die eine Körperoberfläche-Kontaktbereich aufweist; und
eine Basisschicht (2) aus gewirktem Gewebe,
wobei die Maschendichte der Körperkontaktschicht (1) höher ist als die der Basisschicht (2) und wobei die Basisschicht (2) mit nativen Fäden einer ersten Stärke (3) gewirkt ist, **dadurch gekennzeichnet, dass** die Basisschicht mit einem zusätzlichen Faden gewirkt ist, der während des Wirkvorgangs zu der Basisschicht eingeführt wird, um einen lokalisierten Bereich (4) zu bilden, der eine zweite Stärke aufweist, die größer als die erste Stärke ist, und der lokalisierte Bereich (4) mit dem Körperoberfläche-Kontaktbereich (1) deckungsgleich ist, um die Körperkontaktschicht (1) in die Bildung einer erhöhten Außenfläche zum Hervorstehen gegen eine Körperoberfläche zu zwingen.

2. Kontaktsensor (0) nach Anspruch 1, wobei die Körperkontaktschicht (1) ein gewirktes leitfähiges Gewebe ist.

3. Kontaktsensor (0) nach einem vorherigen Anspruch, wobei der Kontaktsensor als ein Element mittels 3D-Wirken hergestellt wird.

4. Kontaktsensor (0) nach Anspruch 1, wobei die Breite des Kontaktsensors 6 mm oder weniger ist.

5. Kontaktsensor (0) nach Anspruch 4, wobei die Breite des Kontaktsensors 3 mm oder weniger ist.

6. Kontaktsensor (0) nach einem vorherigen Anspruch, wobei die Körperkontaktlänge der Körperkontaktschicht (1) mindestens 60 mm ist.

7. Kontaktsensor (0) nach einem vorherigen Anspruch, wobei die Körperkontaktlänge der Körperkontaktschicht (1) mindestens 100 mm ist.

8. Kontaktsensor (0) nach einem vorherigen Anspruch, wobei ferner eine Glasfaser (9) zum Aufnehmen und Übertragen von Doppler-Signalen aus dem Körper eines Trägers bereitgestellt ist.

9. Kontaktsensor (0) nach Anspruch 8, wobei die Glasfaser in einen Kanal eingesetzt ist, der zwischen der Körperkontaktschicht und der Basisschicht gebildet ist.

10. Kontaktsensor (0) nach einem vorherigen Anspruch, wobei eine von der Körperkontaktschicht (1), der Basisschicht (2) oder einer zusätzlich bereitgestellten Schicht (22) hydrophob oder feuchtigkeitsundurchlässig ist und angeordnet ist, um zu verhindern, dass Feuchtigkeit die Körperkontaktschicht berührt.

11. Kontaktsensor (0) nach einem der Ansprüche 1 bis 9, wobei eine von der Körperkontaktschicht, Basisschicht oder einer zusätzlich vorgesehenen Schicht hydrophil oder anderweitig zur Übertragung von Feuchtigkeit geeignet ist, um bei Vorhandensein in der Verwendung aktiv Feuchtigkeit in die Körperkontaktschicht einzuführen.

## Revendications

1. Capteur de contact (0) destiné à surveiller une activité au niveau d'une surface corporelle, comprenant :
une couche en contact avec le corps (1) de tissu à mailles ayant une zone en contact avec la surface corporelle ;
et une couche de base (2) de tissu à mailles, dans lequel la densité de points de la couche en contact avec le corps (1) est supérieure à celle de la couche de base (2) et dans lequel la couche de base (2) est tricotée avec des fils natifs d'une première épaisseur (3), **caractérisé en ce que** la couche de base est tricotée avec un fil supplémentaire introduit dans la couche de base pendant le processus de tricotage pour former une zone localisée (4) qui a une deuxième épaisseur supérieure à la première épaisseur et la zone localisée (4) est congruente avec la zone en contact avec la surface corporelle de la couche en contact avec le corps (1) de manière à pousser la couche en contact avec le corps (1) dans la formation d'une surface externe relevée pour venir en saillie contre une surface corporelle.

2. Capteur de contact (0) selon la revendication 1, dans lequel la couche en contact avec le corps (1) est un tissu conducteur à mailles.

3. Capteur de contact (0) selon l'une quelconque revendication précédente, dans lequel le capteur de contact est produit sous la forme d'un même élément par tricotage 3D.

4. Capteur de contact (0) selon la revendication 1, dans lequel la largeur du capteur de contact est de 6 mm ou moins.

5. Capteur de contact (0) selon la revendication 4, dans lequel la largeur du capteur de contact est de 3 mm ou moins.

6. Capteur de contact (0) selon l'une quelconque revendication précédente, dans lequel la longueur en contact avec le corps de la couche en contact avec le corps (1) est d'au moins 60 mm.

7. Capteur de contact (0) selon l'une quelconque revendication précédente, dans lequel la longueur en contact avec le corps de la couche en contact avec le corps (1) est d'au moins 100 mm.

8. Capteur de contact (0) selon l'une quelconque revendication précédente, dans lequel il est en outre prévu une fibre optique (9) pour le captage et la transmission de signaux Doppler depuis le corps d'un porteur.

9. Capteur de contact (0) selon la revendication 8, dans lequel la fibre optique est ajustée à l'intérieur d'un canal formé entre la couche en contact avec le corps et la couche de base.

10. Capteur de contact (0) selon l'une quelconque revendication précédente, dans lequel l'une de la couche en contact avec le corps (1), la couche de base (2) ou une couche prévue en supplément (22) est hydrophobe ou imperméable à l'humidité et agencée de manière à empêcher l'humidité d'entrer en contact avec la couche en contact avec le corps.

11. Capteur de contact (0) selon l'une quelconque des revendications 1 à 9, dans lequel l'une de la couche en contact avec le corps, la couche de base ou une couche prévue en supplément est hydrophile ou autrement appropriée pour la transmission d'humidité de manière à introduire activement l'humidité, lorsqu'elle est présente en utilisation, vers la couche en contact avec le corps.
